(19) Europäisches Patentamt
European Patent Office
Office européen des brevets

(11) **EP 3 251 703 A1**

(12) **EUROPEAN PATENT APPLICATION**
published in accordance with Art. 153(4) EPC

(43) Date of publication:
06.12.2017 Bulletin 2017/49

(51) Int Cl.:
*A61L 27/00* (2006.01)   *C08F 220/02* (2006.01)
*G02C 7/04* (2006.01)

(21) Application number: 16743407.5

(22) Date of filing: 27.01.2016

(86) International application number:
**PCT/JP2016/052293**

(87) International publication number:
**WO 2016/121804 (04.08.2016 Gazette 2016/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.01.2015 JP 2015015774**

(71) Applicant: **Menicon Co., Ltd.**
**Nagoya-shi**
**Aichi 460-0006 (JP)**

(72) Inventors:
• **NOMURA, Hiroko**
**Kasugai-shi**
**Aichi 487-0032 (JP)**
• **NIWA, Kazuharu**
**Kasugai-shi**
**Aichi 487-0032 (JP)**
• **SUGANUMA, Yuya**
**Kasugai-shi**
**Aichi 487-0032 (JP)**

(74) Representative: **Cabinet Plasseraud**
**66, rue de la Chaussée d'Antin**
**75440 Paris Cedex 09 (FR)**

(54) **MATERIAL FOR INTRAOCULAR LENSES AND METHOD FOR PRESERVING MATERIAL FOR INTRAOCULAR LENSES**

(57) The intraocular lens material according to the present invention includes a polymer produced by polymerizing a polymerizable composition including a hydrophilic monomer and a crosslinkable monomer. The A×B value that is the product of the molar fraction A of the hydrophilic monomer and the molar fraction B of the crosslinkable monomer is more than $2.1 \times 10^{-3}$ and less than $3.6 \times 10^{-3}$. The molar fraction B of the crosslinkable monomer is less than $2.4 \times 10^{-2}$. The polymer has a refractive index n of 1.5 or more in a state of hydration.

**Description**

Technical Field

The present invention relates to an intraocular lens material and a method for preserving an intraocular lens material.

Background Art

Soft materials that are flexible and foldable and suitably used for producing intraocular lenses have been developed with the development of small-incision cataract surgery. Acrylic materials, which have a high refractive index and slowly unfold after being implanted into an eye, are particularly desirable. When the shape recoverability of a lens is enhanced, the elongation percentage of the lens is reduced. In such a case, the material can be brittle and easily torn when flaws or the like are present thereon. On the other hand, in order to implant a lens into an eye through a minimum incision, it is desirable to use a material having a large elongation percentage and resistance to cracking and tearing.

One of such intraocular lens materials proposed in the related art is an intraocular lens material that includes a polymer produced by polymerizing a polymerizable component including a hydrophilic monomer including a hydroxyl group-containing alkyl (meth)acrylate, a (meth)acrylamide monomer, and an N-vinyl lactam, the intraocular lens material having a water absorption percentage of 1.5% to 4.5% by mass (see, e.g., PTL 1). The above intraocular lens material, which has excellent flexibility and a high refractive index, allows the thickness of a lens to be reduced, and enables a lens to be implanted through an incision while folded. The above intraocular lens material also has excellent transparency and minimizes the occurrence of glistening.

Citation List

Patent Literature

PTL 1: Japanese Unexamined Patent Application Publication No. 11-56998

Summary of the Invention

Technical Problem

Although the intraocular lens material described in PTL 1 has improved functions by including the hydrophilic monomer, there has been a demand for further improvement of the functions of the intraocular lens material.

The present invention was made in light of the above-described issue. It is a primary object of the present invention to provide an intraocular lens material having better flexibility and a higher strength and capable of reducing the occurrence of glistening and a method for preserving the intraocular lens material.

Solution to Problem

The inventors of the present invention conducted extensive studies in order to address the above-described issue and, as a result, found that setting the molar fractions of a hydrophilic monomer and a crosslinkable monomer included in an intraocular lens material to fall within predetermined preferable ranges further enhances the flexibility and strength of the intraocular lens material and reduces the occurrence of glistening. Thus, the present invention was made.

The intraocular lens material according to the present invention includes a polymer produced by polymerizing a polymerizable composition including a hydrophilic monomer and a crosslinkable monomer, wherein an A×B value is more than $2.1 \times 10^{-3}$ and less than $3.6 \times 10^{-3}$, the A×B value being the product of a molar fraction A of the hydrophilic monomer and a molar fraction B of the crosslinkable monomer, the molar fraction B of the crosslinkable monomer is less than $2.4 \times 10^{-2}$, and the polymer has a refractive index n of 1.5 or more in a state of hydration.

The method for preserving the intraocular lens material according to the present invention includes enclosing the above-described intraocular lens material and a moistened humidity-controlling material in a moisture-proof package.

The present invention provides an intraocular lens material having better flexibility and a higher strength and capable of reducing the occurrence of glistening and a method for preserving the intraocular lens material. The reasons for which the above advantageous effects are achieved are presumably, for example, as follows. When a lens made of, for example, an acrylic material is implanted into an eye or used in a physiological environment, small bright spots are commonly formed in the lens as a result of glistening (condensation of water molecules in the lens). It is desirable that intraocular lenses have a large elongation percentage, because they are implanted into an eye through a minimum incision while folded. While reducing the crosslinking density in a lens is effective for increasing the elongation percentage of the lens, the reduction in crosslinking density also increases the occurrence of glistening. Accordingly, the occurrence of glistening may be reduced by increasing the water absorption percentage of the lens. In the present invention, the $A \times B$ value that is the product of the molar fraction A of the hydrophilic monomer and the molar fraction B of the crosslinkable monomer is more than $2.1 \times 10^{-3}$ and less than $3.6 \times 10^{-3}$, and the molar fraction B of the crosslinkable monomer is less than $2.4 \times 10^{-2}$. This further enhances the flexibility and strength of the intraocular lens material and reduces the occurrence of glistening. It was also confirmed that the refractive index of the lens is not significantly reduced even when the water absorption percentage of the lens is high, although an increase in the water absorption percentage of a lens is considered to reduce the refractive index of the lens implanted in an eye (in a physiological environment). Although increasing the water absorption percentage of an intraocular lens material (increasing the amount of hydrophilic component) commonly reduces the flexibility of the intraocular lens material, it is possible to control the flexibility of the intraocular lens material by enclosing the intraocular lens material and a humidity-controlling material in a moisture-proof package.

Brief Description of Drawings

Fig. 1 is a diagram illustrating an example of a moisture-proof package 10 according to the embodiment.
Fig. 2 is a diagram illustrating a specimen used for the measurement of elongation percentage.

Description of Embodiments

The intraocular lens material according to the present invention includes a polymer produced by polymerizing a polymerizable composition including a hydrophilic monomer and a crosslinkable monomer. The $A \times B$ value that is the product of the molar fraction A of the hydrophilic monomer and the molar fraction B of the crosslinkable monomer is more than $2.1 \times 10^{-3}$ and less than $3.6 \times 10^{-3}$. The molar fraction B of the crosslinkable monomer is less than $2.4 \times 10^{-2}$. The polymer has a refractive index n of 1.5 or more in a state of hydration. The polymerizable composition included in the intraocular lens material preferably includes, as main components, an aromatic-ring-containing (meth)acrylate and an alkyl (meth)acrylate including an alkyl group having 1 to 20 carbon atoms. That is, the polymerizable composition used as a raw material of the intraocular lens material may include at least the main component monomers, the hydrophilic monomer, and the crosslinkable monomer.

The aromatic-ring-containing (meth)acrylate, which serves as a main component monomer, may be a component that increases the refractive index of the intraocular lens material. Examples of the aromatic-ring-containing (meth)acrylate include phenoxyethyl (meth)acrylate, phenylethyl (meth)acrylate, benzyl (meth)acrylate, phenyl (meth)acrylate, and pentabromophenyl (meth)acrylate. The above aromatic-ring-containing (meth)acrylates may be used alone or in a mixture of two or more. In order to markedly increase the refractive index of the intraocular lens material, one or more selected from phenoxyethyl acrylate, phenylethyl acrylate, and benzyl acrylate are preferably used. In order to further enhance the flexibility of the intraocular lens material, phenoxyethyl acrylate is particularly preferably used. The proportion of the amount of the aromatic-ring-containing (meth)acrylate to the total amount of main components is preferably 15% by mass or more and 80% by mass or less and may be 50% by mass or more and 70% by mass or less.

The alkyl (meth)acrylate including an alkyl group having 1 to 20 carbon atoms, which serves as a main component monomer, may be a component that enhances the shape recoverability and flexibility of the intraocular lens material. Examples of the alkyl (meth)acrylate include straight-chain, branched-chain, or cyclic alkyl (meth)acrylates, such as methyl acrylate, ethyl acrylate, propyl acrylate, butyl acrylate, pentyl acrylate, hexyl acrylate, heptyl acrylate, nonyl acrylate, stearyl (meth)acrylate, octyl (meth)acrylate, decyl (meth)acrylate, lauryl (meth)acrylate, pentadecyl (meth)acrylate, 2-ethylhexyl (meth)acrylate, cyclopentyl acrylate, and cyclohexyl acrylate; and fluorine-containing alkyl (meth)acrylates, such as 2,2,2-trifluoroethyl (meth)acrylate, 2,2,3,3-tetrafluoropropyl (meth)acrylate, 2,2,3,3-tetrafluoro-t-pentyl (meth)acrylate, 2,2,3,4,4,4-hexafluorobutyl (meth)acrylate, 2,2,3,4,4,4-hexafluoro-t-hexyl (meth)acrylate, 2,3,4,5,5,5-hexafluoro-2,4-bis(trifluoromethyl)pentyl (meth)acrylate, 2,2,3,3,4,4-hexafluorobutyl (meth)acrylate, 2,2,2,2',2',2'-hexafluoroisopropyl (meth)acrylate, 2,2,3,3,4,4,4-heptafluorobutyl (meth)acrylate, and 2,2,3,3,4,4,5,5-octafluoropentyl (meth)acrylate. The above alkyl (meth)acrylates may be used alone or in a mixture of two or more. In order to markedly enhance the shape recoverability and flexibility of the intraocular lens material, an alkyl acrylate including an alkyl group having 1 to 5 carbon atoms is preferably used, and ethyl acrylate and butyl acrylate are particularly preferably used. The proportion of the amount of the alkyl (meth)acrylate to the total amount of main components is preferably 20% by mass or more and 85% by mass or less and may be 30% by mass or more and 50% by mass or less.

The hydrophilic monomer included in the polymerizable composition is a component that imparts hydrophilicity to the intraocular lens material. The hydrophilic monomer may also be a component that promotes a reduction in the likelihood of glistening occurring in the intraocular lens material. The hydrophilic monomer may include, for example, one or more selected from a hydroxyl group-containing alkyl (meth)acrylate including an alkyl group having 1 to 20 carbon atoms, a (meth)acrylamide monomer, and an N-vinyl lactam. The polymerizable composition may further include a hydrophilic monomer other than the above hydrophilic monomers. Examples of the hydroxyl group-containing alkyl (meth)acrylate include hydroxyalkyl (meth)acrylates, such as hydroxyethyl (meth)acrylate, hydroxypropyl (meth)acrylate, hydroxybutyl (meth)acrylate, and hydroxypentyl (meth)acrylate. Examples of the hydroxyl group-containing alkyl (meth)acrylate also include dihydroxyalkyl (meth)acrylates, such as dihydroxypropyl (meth)acrylate, dihydroxybutyl (meth)acrylate, and dihydroxypentyl (meth)acrylate. Examples of the (meth)acrylamide monomer include N,N-dialkyl (meth)acrylamides, such as N,N-dimethyl (meth)acrylamide, N,N-diethyl (meth)acrylamide, and N,N-dipropyl (meth)acrylamide. Examples of the (meth)acrylamide monomer also include N,N-dialkylaminoalkyl (meth)acrylamides, such as N,N-dimethylaminopropyl (meth)acrylamide and N,N-diethylaminopropyl (meth)acrylamide. Examples of the N-vinyl lactam include N-vinylpyrrolidone, N-vinylpiperidone, and N-vinylcaprolactam. Examples of the other hydrophilic monomer include diethylene glycol mono(meth)acrylate, triethylene glycol mono(meth)acrylate, propylene glycol mono(meth)acrylate, (meth)acrylic acid, 1-methyl-3-methylene-2-pyrrolidinone, maleic anhydride, maleic acid, maleic acid derivatives, fumaric acid, fumaric acid derivatives, aminostyrene, and hydroxystyrene. The above hydrophilic monomers may be used alone or in a mixture of two or more.

Among the above hydrophilic monomers, in particular, the hydroxyl group-containing alkyl (meth)acrylate and the (meth)acrylamide monomer are preferable since they are capable of markedly promoting a reduction in the occurrence of glistening. The molar fraction A of the hydrophilic monomer in the entire polymerizable composition is preferably more than $0.9 \times 10^{-1}$ and is more preferably $1.7 \times 10^{-1}$ or more. The molar fraction A of the hydrophilic monomer is preferably less than $3.6 \times 10^{-1}$ and more preferably $2.8 \times 10^{-1}$ or less. The amount of the hydrophilic monomer included in the polymerizable composition is preferably 15 parts by mass or more and 25 parts by mass or less and is more preferably 20 parts by mass or more relative to 100 parts by mass of the main component. When the content of the hydrophilic monomer in the polymerizable composition falls within the above range, the hydrophilic monomer sufficiently promotes a reduction in the occurrence of glistening.

The crosslinkable monomer included in the polymerizable composition may be a component that controls the flexibility of the intraocular lens material, increases the mechanical strength of the intraocular lens material, and enhances the shape recoverability of the intraocular lens material and the copolymerizability between the polymerizable components, such as the hydrophilic monomer and other polymerizable monomers. Examples of the crosslinkable monomer include butanediol di(meth)acrylate, ethylene glycol di(meth)acrylate, diethylene glycol di(meth)acrylate, triethylene glycol di(meth)acrylate, propylene glycol di(meth)acrylate, dipropylene glycol di(meth)acrylate, diallyl fumarate, allyl (meth)acrylate, vinyl (meth)acrylate, trimethylolpropane tri(meth)acrylate, methacryloyloxyethyl (meth)acrylate, divinylbenzene, diallyl phthalate, diallyl adipate, triallyl diisocyanate, $\alpha$-methylene-N-vinylpyrrolidone, 4-vinylbenzyl (meth)acrylate, 3-vinylbenzyl (meth)acrylate, 2,2-bis((meth)acryloyloxyphenyl)hexafluoropropane, 2,2-bis((meth)acryloyloxyphenyl)propane, 1,4-bis(2-(meth)acryloyloxyhexafluoroisopropyl)benzene, 1,3-bis(2-(meth)acryloyloxyhexafluoroisopropyl)benzene, 1,2-bis(2-(meth)acryloyloxyhexafluoroisopropyl)benzene, 1,4-bis(2-(meth)acryloyloxyisopropyl)benzene, 1,3-bis(2-(meth)acryloyloxyisopropyl)benzene, and 1,2-bis(2-(meth)acryloyloxyisopropyl)benzene. The above crosslinkable monomers may be used alone or in a mixture of two or more.

Among the above crosslinkable monomers, in particular, one or more selected from butanediol di(meth)acrylate and ethylene glycol di(meth)acrylate are preferable, because they are capable of controlling the flexibility of the intraocular lens material, increasing the mechanical strength of the intraocular lens material, and enhancing the shape recoverability and copolymerizability of the intraocular lens material in a further suitable manner. The molar fraction B of the crosslinkable monomer in the entire polymerizable composition is less than $2.4 \times 10^{-2}$. The molar fraction B of the crosslinkable monomer is preferably less than $2.1 \times 10^{-2}$, is more preferably $1.7 \times 10^{-2}$ or less, and is further preferably less than $1.6 \times 10^{-2}$. The molar fraction B of the crosslinkable monomer is preferably $1.0 \times 10^{-2}$ or more and is more preferably $1.1 \times 10^{-2}$ or more. The amount of the crosslinkable monomer included in the polymerizable composition is preferably 1 part by mass or more and 3 parts by mass or less and is more preferably 1 part by mass or more and 2 parts by mass or less relative to 100 parts by mass of the main component. When the content of the crosslinkable monomer falls within the above range, the flexibility of the intraocular lens material is further enhanced.

The polymerizable composition may include other additional components, such as an ultraviolet absorber and a coloring agent. Examples of the ultraviolet absorber include benzophenones, such as 2-hydroxy-4-methoxybenzophenone and 2-hydroxy-4-octoxybenzophenone; benzotriazoles, such as 2-(2'-hydroxy-5'-methacryloxyethyleneoxy-t-butylphenyl)-5-methylbenzotriazole, 2-(2'-hydroxy-5'-methylphenyl)benzotriazole, and 5-chloro-2(3'-t-butyl-2'-hydroxy-5'-methylphenyl)benzotriazole; salicylic acid derivatives; and hydroxyacetophenone derivatives. The amount of the ultraviolet absorber is preferably, for example, 0.05 parts by mass or more and 3 parts by mass or less relative to 100 parts by mass of the total amount of polymerizable composition. A yellow or orange coloring agent is desirably used for correcting cyanopsia or the like. Examples of the coloring agent include the following coloring agents described in Color Index (CI): oil-soluble dyes, such as CI Solvent Yellow and CI Solvent Orange; dispersion dyes, such as CI Disperse Yellow and CI Disperse Orange; and vat dyes. The amount of the coloring agent is preferably 0.01 parts by mass or more and 3 parts by mass or less relative to 100 parts by mass of the total amount of polymerizable composition.

A radical polymerization initiator, a photopolymerization initiator, and the like may be added to the polymerizable composition for performing polymerization. The polymerization method may be, for example, a method in which a radical polymerization initiator is added to the polymerizable composition and the polymerizable composition is subsequently heated or a method in which the polymerizable composition is irradiated with electromagnetic wave, such as microwave, ultraviolet radiation, or radiation ($\gamma$ radiation). Examples of the radical polymerization initiator include azobisisobutyronitrile, azobisdimethylvaleronitrile, benzoyl peroxide, t-butyl hydroperoxide, and cumene hydroperoxide. In the case where a light beam or the like is used for performing polymerization, it is preferable to further add a photopolymerization initiator or a sensitizer to the polymerizable composition. Examples of the photopolymerization initiator include benzoin compounds, such as methyl ortho-benzoyl benzoate; phenone compounds, such as 2-hydroxy-2-methyl-1-phenylpropane-1-one; thioxanthone compounds, such as 1-hydroxycyclohexyl phenyl ketone, 1-phenyl-1,2-propanedione-2-(o-ethoxycarbonyl) oxime, and 2-chlorothioxanthone; dibenzosuberone; 2-ethylanthraquinone; benzophenone acrylate; benzophenone; and benzil. The amount of polymerization initiator or sensitizer used is preferably 0.01 parts by mass or more and 2 parts by mass or less relative to 100 parts by mass of the total amount of polymerizable composition in order to conduct the polymerization reaction at a sufficient rate. For forming the intraocular lens material according to the present invention into an intraocular lens, for example, lathing or molding may be employed.

In the intraocular lens material according to the present invention, the product of the molar fraction A of the hydrophilic monomer and the molar fraction B of the crosslinkable monomer, that is, the A×B value, is more than $2.1 \times 10^{-3}$ and less than $3.6 \times 10^{-3}$, and the molar fraction B of the crosslinkable monomer is less than $2.4 \times 10^{-2}$. When the A×B value and the molar fraction B of the crosslinkable monomer fall within the above respective ranges, the intraocular lens material has better flexibility and a further high strength. Moreover, the occurrence of glistening is further reduced. The A×B value is preferably $2.2 \times 10^{-3}$ or more and less than $3.5 \times 10^{-3}$.

The intraocular lens material according to the present invention has a refractive index n of 1.5 or more in a state of hydration. In the intraocular lens material according to the present invention, where the A×B value is more than $2.1 \times 10^{-3}$ and less than $3.6 \times 10^{-3}$ and the molar fraction B of the crosslinkable monomer is less than $2.4 \times 10^{-2}$, a reduction in refractive index between the dried state and the moistened state is further limited.

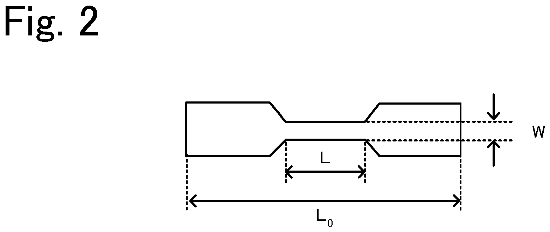

The elongation percentage of the intraocular lens material according to the present invention is preferably 290% or more and is more preferably 300% or more. When the elongation percentage of the intraocular lens material is 290% or more, the flexibility of the intraocular lens material may be further enhanced. The elongation percentage of the intraocular lens material is preferably 600% or less in consideration of the shape recoverability of the intraocular lens material. The elongation percentage (%) of the intraocular lens material is determined by immersing a dumbbell-shaped specimen (see Fig. 2 below) in water having a constant temperature of 25°C, leaving the specimen to stand for 1 minute, subjecting the specimen to a tension at a speed of 100 mm/min until the specimen ruptures, and measuring the strain when the maximum load occurs in the tensile test.

The glass transition temperature (Tg) of the intraocular lens material according to the present invention is preferably 30°C or less, is more preferably 25°C or less, and is further preferably 20°C or less. When the glass transition temperature (Tg) of the intraocular lens material is 30°C or less or, in particular, 20°C or less, the performances of the lens which are required when the lens is folded and unfolded inside an eye are not likely to be adversely affected, which is preferable.

The water absorption percentage (mass%) of the intraocular lens material according to the present invention is preferably 2.2% by mass or more and 4.5% by mass or less. When the water absorption percentage of the intraocular lens material is 2.2% by mass or more, the occurrence of glistening may be reduced. When the water absorption percentage of the intraocular lens material is 4.5% by mass or less, the reduction in the flexibility of the intraocular lens material and the reduction in the shape recoverability of the intraocular lens material may further limited. Since the above-described intraocular lens material according to the present invention has excellent flexibility and a high refractive index, the thickness of a lens may be reduced. This makes it possible to implant a lens into an eye through a small incision while folded. The above-described intraocular lens material according to the present invention also has excellent transparency and enables the occurrence of glistening to be reduced.

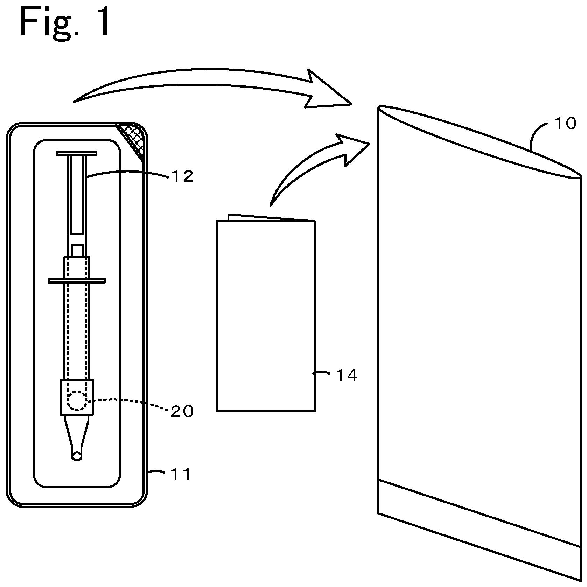

A method for preserving the intraocular lens material according to the present invention is a method including enclosing any one of the above-described intraocular lens materials and a moistened humidity-controlling material in a moisture-proof package. The humidity-controlling material used in this preservation method is preferably a humidified package insert (paper). In such a case, the package insert also serves as a humidity-controlling material. This enables the humidity atmosphere in the moisture-proof package to be controlled with a simple structure. The humidity-controlling material is not limited to paper and may also be a moistened polymer material. In such a case, the amount of water absorbed in the humidity-controlling material may be further increased. The moisture-proof package is preferably a package having a moisture permeability of 0.1 g/(m$^2$·day) or less (40°C, 90%RH), such as an aluminum-laminated bag or a TECHBARRIER (registered trademark). Fig. 1 illustrates an example of a moisture-proof package 10. As illustrated in Fig. 1, the moisture-proof package 10 contains an intraocular lens 20 made of the intraocular lens material according to the present invention, an injector device 12 preloading the intraocular lens 20, and a package insert 14 made of paper, which provides instructions for handling the intraocular lens. The injector device 12 preloading the intraocular lens 20 is housed in a sterilized package 11. The package insert 14 may be an Instructions for Use for the injector device 12 and the intraocular lens 20. The amount of moisture absorbed in the package insert 14 may be set appropriately on the basis of, for example, the flexibility and maximum water absorption percentage of the intraocular lens 20. For causing the humidity-controlling material to absorb moisture, for example, the humidity-controlling material may be charged into a thermo-hygrostat having a predetermined temperature (e.g., room temperature) and a predetermined relative humidity (e.g., 40% to 70%RH). Alternatively, water droplets may be dropped onto the humidity-controlling material. When the intraocular lens 20 has a large maximum water absorption percentage, it is preferable to increase the amount of water (moisture) absorbed in the humidity-controlling material. Preserving the intraocular lens in the above-described manner enables the humidity inside the moisture-proof package to be maintained. This makes it easy to bend even an intraocular lens made of a material that is resistant to bending when dried and enables the intraocular lens to be stored and used in the injector device.

In the intraocular lens material according to the embodiment and the method for preserving the intraocular lens material which are described above in detail, the A×B value that is the product of the molar fraction A of the hydrophilic monomer and the molar fraction B of the crosslinkable monomer is more than $2.1 \times 10^{-3}$ and less than $3.6 \times 10^{-3}$, and the molar fraction B of the crosslinkable monomer is less than $2.4 \times 10^{-2}$. This enables the flexibility and strength of the intraocular lens material to be further enhanced and the occurrence of glistening to be reduced. This is presumably because the reduction in crosslinking density increases the elongation percentage of the intraocular lens material and the occurrence of glistening, which may be caused as a result of the reduction in crosslinking density, can be reduced by increasing the water absorption percentage in the intraocular lens material.

The present invention is not limited to the above-described embodiment, and can be carried out by various modes as long as they belong to the technical scope of the invention.

For example, although the intraocular lens 20 described in the above embodiment is stored in the injector device 12, the present invention is not limited to this. The intraocular lens 20 may be stored in the moisture-proof package together with the humidity-controlling material instead of being stored in the injector device. The sterilized package 11 may be omitted.

Examples

Experimental Examples in which the intraocular lenses according to the present invention were prepared specifically are described below. Experimental Examples 3, 5, 8, 10, 13, 14, 15, 18, 19, and 20 correspond to Examples of the present invention. Experimental Examples 1, 2, 4, 6, 7, 9, 11, 12, 16, 17, and 21 to 23 correspond to Comparative Examples. Needless to say that the present invention is not limited by Examples below and may be implemented in various forms within the technical scope of the present invention.

[Components Used]

The abbreviations for the compounds used in Experimental Examples are described below.

<Main Components>

POEA: 2-Phenoxyethyl acrylate Molecular weight 192.21
EA : Ethyl acrylate Molecular weight 100.12

<Hydrophilic Monomers>

HEMA: 2-Hydroxyethyl methacrylate Molecular weight 130.15
DMAA: N,N-Dimethylacrylamide Molecular weight 99.14
NVP : N-Vinyl-2-pyrrolidone Molecular weight 111.14

<Crosslinkable Monomers>

BDDA: 1,4-Butanediol diacrylate Molecular weight 198.22
EDMA: Ethylene glycol dimethacrylate Molecular weight 198.22

[Preparation of Intraocular Lens Materials]

Each of the sets of polymerizable components described in Table 1 was mixed with 0.5 parts by mass of 2,2'-azobis(2,4-dimethyl valeronitrile) used as a polymerization initiator relative to 100 parts by mass of the main components. The resulting mixtures were each charged into a mold having a desired shape. The molds were put into an oven and heat polymerization molding was carried out at 80°C for 40 minutes. The resulting polymers were demolded. After an elution treatment had been performed, the polymers were dried at 60°C for 2 days. Hereby, intraocular lens materials were prepared.

[Table 1]

| Specimen | Main component | | Hydrophilic monomer | | | Crosslinkable monomer | |
|---|---|---|---|---|---|---|---|
| | POEA | EA | HEMA | DMAA | NVP | BDDA | EDMA |
| Experimental Example 1 | 60 | 40 | 15 | - | - | 4 | - |
| Experimental Example 2 | 60 | 40 | 15 | - | - | 2 | - |
| Experimental Example 3 | 60 | 40 | 20 | - | - | 3 | - |
| Experimental Example 4 | 60 | 40 | 20 | - | - | 2 | - |
| Experimental Example 5 | 60 | 40 | 25 | - | - | 2 | - |
| Experimental Example 6 | 60 | 40 | 15 | - | - | - | 4 |
| Experimental Example 7 | 60 | 40 | 15 | - | - | - | 2 |
| Experimental Example 8 | 60 | 40 | 20 | - | - | - | 3 |
| Experimental Example 9 | 60 | 40 | 20 | - | - | - | 2 |
| Experimental Example 10 | 60 | 40 | 25 | - | - | - | 2 |
| Experimental Example 11 | 60 | 40 | - | 15 | - | 4 | - |
| Experimental Example 12 | 60 | 40 | - | 15 | - | 2 | - |
| Experimental Example 13 | 60 | 40 | - | 20 | - | 3 | - |
| Experimental Example 14 | 60 | 40 | - | 20 | - | 2 | - |
| Experimental Example 15 | 60 | 40 | - | 25 | - | 2 | - |
| Experimental Example 16 | 60 | 40 | - | - | 15 | 4 | - |
| Experimental Example 17 | 60 | 40 | - | - | 15 | 2 | - |
| Experimental Example 18 | 60 | 40 | - | - | 20 | 3 | - |
| Experimental Example 19 | 60 | 40 | - | - | 20 | 2 | - |
| Experimental Example 20 | 60 | 40 | - | - | 25 | 2 | - |
| Experimental Example 21 | 60 | 40 | 21 | - | - | - | 2 |
| Experimental Example 22 | 60 | 40 | - | 16 | - | 2 | - |
| Experimental Example 23 | 60 | 40 | - | - | 18 | 2 | - |

<Measurement of Physical Properties>

(Water Absorption Percentage)

The water absorption percentage (mass%) of each of the intraocular lens materials was determined by measuring the mass of the specimen at 25°C when the specimen was in the equilibrium moisture state and the dried state. Specifically, the water absorption percentage of each intraocular lens material was determined from the mass W of the specimen which was measured at 25°C when the specimen was in the equilibrium moisture state and the mass W0 of the specimen which was measured at 25°C when the specimen was in the dried state using the following expression.

$$\text{Water Absorption Percentage (mass\%)} = (W - W0)/W0 \times 100$$

(Glistening)

The specimens were immersed in water having a temperature of 35°C for 24 hours and subsequently in water having a temperature of 25°C for 2 hours. Then, the appearance of each of the specimens was inspected with a stereoscopic microscope. In the inspection of the appearance of each specimen, three samples were taken from the specimen, and the number of spots (white bright spots) at which glistening was present was determined. The specimens used in the above measurement had a +20D lens-like shape having a diameter of 6 mm.

(Refractive Index)

The refractive index of each of the specimens was determined using the Mercury e-line when the specimen was in the dried state (25°C) and the moistened state (35°C) .

(Glass Transition Temperature: Tg)

The specimens were each subjected to differential scanning calorimetry in the range of -30°C to 70°C with the rate of temperature rise of 20 °C/min in order to determine the mid-point glass transition temperature of the specimen.

(Tensile Test)

Specimens used in the tensile test had a dumbbell-shape having a total length (L0) of 20 mm, a parallel-portion length (L) of 6 mm, a parallel-portion width (W) of 1.5 mm, and a thickness of 0.8 mm (see Fig. 2). The specimens were each immersed in water having a constant temperature of 25°C, left to stand for 1 minute, and subjected to a tension at a speed of 100 mm/min until it ruptured. The strain (= elongation percentage (%)) of each specimen at which the maximum load occurred was output using software.

(Compressive Load)

The specimens were each compressed in an environment of 23°C and 50%RH at a compressive speed of 5.0 mm/min, and the load that occurred when the distance between jigs reached 3.0 mm was measured. The specimens used in the above measurement had a +20D lens-like shape having a diameter of 6 mm. Prior to the above measurement, the specimens were each hermetically sealed in an aluminum package under Dry and Wet conditions below in order to control and stabilize the state of the specimen. Dry conditions were conditions where each specimen was enclosed in the aluminum package together with a humidity-controlling material that was a package insert (paper) having a water absorption percentage of 0% by mass which had been dried at 80°C. In Experimental Examples 1, 3, 5, 6, 10, and 11, Wet conditions were conditions where each specimen was enclosed in the aluminum package together with a humidity-controlling material that was a package insert (paper) having a water absorption percentage of 5.1% by mass which had been conditioned at 23°C, 50%RH. In Experimental Examples 15, 16, and 20, Wet conditions were conditions where each specimen was enclosed in the aluminum package together with a humidity-controlling material that was a polymer (soft contact lens material) having a water content of 65.0% by mass.

(Results and Discussion)

Tables 2 and 3 summarize the details of the specimens used in Experimental Examples 1 to 23 and the measurement results. Table 2 summarizes the molar fraction A of the hydrophilic monomer, the molar fraction B of the crosslinkable monomer, the A×B value, water absorption percentage (mass%), the number of glistening spots, refractive indices $n_e$ measured in the dried state and the moistened state, Tg (°C), and elongation percentage (%). In the column of the number of glistening spots, the values of three samples are separated by "/". Table 3 summarizes the A×B value and compressive loads (mN) measured in the Dry and Wet states. As shown in Table 2, the water absorption percentages of the specimens were 1.7% to 4.5% by mass. Furthermore, in all of the specimens, the occurrence of glistening was increased when the product of the molar fraction of the hydrophilic monomer and the molar fraction of the crosslinkable monomer (A×B value) was $2.1 \times 10^{-3}$ or less. On the other hand, when the A×B value was more than $2.1 \times 10^{-3}$ and, in particular, when the A×B value was $2.2 \times 10^{-3}$ or more, the occurrence of glistening was reduced. Note that, it is considered that the likelihood of occurrence of glistening is small when the average of the numbers of glistening spots of 3 samples was 5 or less. It was also confirmed that, although the specimens each included different type of hydrophilic monomers and crosslinkable monomers in different amounts (Table 1), the refractive indices n of each specimen substantially did not vary between the dried state and the moistened state, that is, the refractive indices n of each specimen were both 1.5 or more. The difference between the refractive indices in the dried and moistened states was less than 0.010 even when the water absorption percentage of the specimen was high.

While the elongation percentages of the specimens in which the amount of crosslinkable monomer was 4 parts by mass or more (molar fraction: $2.3 \times 10^{-2}$ or more) relative to 100 parts by mass of the main component were less than 290%, the elongation percentages of the specimens in which the amount of crosslinkable monomer was reduced to 3 parts by mass (molar fraction: $1.7 \times 10^{-2}$ or less) exceeded 290%. It was also confirmed that, when the hydrophilic monomer is DMAA or NVP and the crosslinkable monomer is EDMA, the flexibility of the lens may be reduced as shown by Tg in Table 2 and the compressive load in Table 3. The Tg of the lens is preferably 20°C or less, because it affects the performance of the lens which are required when the lens is folded and unfolded inside an eye. Accordingly, it was found that using the hydrophilic monomer HEMA and the crosslinkable monomer BDDA in combination is more suitable as an intraocular lens material. As shown in Table 3, it was also found that even a material that has a high compressive load or is inflexible in the dried state may have a reduced compressive load or become flexible when a humidity-controlling material having an adequately controlled humidity is enclosed together with the lens material in order to control the moistened state of the lens material. Thus, even materials relatively inflexible in the dried state may be used as intraocular lens materials when they are distributed such that the moisture contents of the materials (humidity inside a package) are maintained at an adequate level.

[Table 2]

| Specimen | Molar fraction A of hydrophilic monomer [1] | Molar fraction B of crosslinkable monomer [2] | A×B | Water absorption percent -age mass % | Number of glistening spots[3] | Refractive index n | | Tg °C | Elongation percent -age % |
|---|---|---|---|---|---|---|---|---|---|
| | | | | | | Dried | Moistened | | |
| Experimental Example 1 | $1.4 \times 10^{-1}$ | $2.4 \times 10^{-2}$ | $3.2 \times 10^{-3}$ | 2.0 | 0/3/1 | 1.524 | 1.519 | 13 | 230 |
| Experimental Example 2 | $1.4 \times 10^{-1}$ | $1.2 \times 10^{-2}$ | $1.7 \times 10^{-3}$ | 1.9 | */*/* | 1.523 | 1.518 | 11 | - |
| Experimental Example 3 | $1.7 \times 10^{-1}$ | $1.7 \times 10^{-2}$ | $3.0 \times 10^{-3}$ | 2.2 | 2/0/1 | 1.524 | 1.518 | 12 | 297 |
| Experimental Example 4 | $1.8 \times 10^{-1}$ | $1.2 \times 10^{-2}$ | $2.0 \times 10^{-3}$ | 2.2 | 6/9/10 | 1.524 | 1.517 | 12 | - |
| Experimental Example 5 | $2.1 \times 10^{-1}$ | $1.1 \times 10^{-2}$ | $2.3 \times 10^{-3}$ | 2.8 | 3/1/0 | 1.522 | 1.516 | 13 | 492 |
| Experimental Example 6 | $1.4 \times 10^{-1}$ | $2.4 \times 10^{-2}$ | $3.2 \times 10^{-3}$ | 2.1 | 6/1/1 | 1.526 | 1.519 | 14 | 262 |
| Experimental Example 7 | $1.4 \times 10^{-1}$ | $1.2 \times 10^{-2}$ | $1.7 \times 10^{-3}$ | 1.7 | */*/* | 1.525 | 1.520 | 11 | - |
| Experimental Example 8 | $1.7 \times 10^{-1}$ | $1.7 \times 10^{-2}$ | $3.0 \times 10^{-3}$ | 2.2 | 0/2/0 | 1.524 | 1.518 | 13 | - |
| Experimental Example 9 | $1.8 \times 10^{-1}$ | $1.2 \times 10^{-2}$ | $2.0 \times 10^{-3}$ | 2.4 | */*/* | 1.523 | 1.518 | 12 | - |
| Experimental Example 10 | $2.1 \times 10^{-1}$ | $1.1 \times 10^{-2}$ | $2.3 \times 10^{-3}$ | 3.1 | 3/2/2 | 1.524 | 1.517 | 12 | 495 |
| Experimental Example 11 | $1.7 \times 10^{-1}$ | $2.3 \times 10^{-2}$ | $39 \times 10^{-3}$ | 2.5 | 0/1/1 | 1.527 | 1.522 | 20 | 242 |
| Experimental Example 12 | $1.7 \times 10^{-1}$ | $1.2 \times 10^{-2}$ | $2.0 \times 10^{-3}$ | 2.6 | 15/12/8 | 1.528 | 1.522 | 16 | - |
| Experimental Example 13 | $2.2 \times 10^{-1}$ | $1.6 \times 10^{-2}$ | $3.5 \times 10^{-3}$ | 3.4 | 0/0/1 | 1.527 | 1.521 | 20 | - |
| Experimental Example 14 | $2.2 \times 10^{-1}$ | $1.1 \times 10^{-2}$ | $2.4 \times 10^{-3}$ | 3.4 | 0/0/0 | 1.527 | 1.522 | 19 | - |

EP 3 251 703 A1

(continued)

| Specimen | Molar fraction A of hydrophilic monomer [1] | Molar fraction B of crosslinkable monomer [2] | A×B | Water absorption percent -age mass % | Number of glistening spots[3] | Refractive index n | | Tg °C | Elongation percent -age % |
|---|---|---|---|---|---|---|---|---|---|
| | | | | | | Dried | Moistened | | |
| Experimental Example 15 | $2.6 \times 10^{-1}$ | $1.0 \times 10^{-2}$ | $2.7 \times 10^{-3}$ | 4.3 | 0/0/0 | 1.527 | 1.520 | 24 | 516 |
| Experimental Example 16 | $1.6 \times 10^{-1}$ | $2.3 \times 10^{-2}$ | $3.6 \times 10^{-3}$ | 2.7 | 1/1/0 | 1.531 | 1.525 | 23 | 286 |
| Experimental Example 17 | $1.6 \times 10^{-1}$ | $1.2 \times 10^{-2}$ | $1.9 \times 10^{-3}$ | 2.7 | 5/*/* | 1.531 | 1.525 | 19 | - |
| Experimental Example 18 | $2.0 \times 10^{-1}$ | $1.7 \times 10^{-2}$ | $3.3 \times 10^{-3}$ | 3.8 | 0/0/0 | 1.532 | 1.526 | 24 | - |
| Experimental Example 19 | $2.0 \times 10^{-1}$ | $1.1 \times 10^{-2}$ | $2.2 \times 10^{-3}$ | 3.4 | 0/0/0 | 1.532 | 1.525 | 22 | - |
| Experimental Example 20 | $2.4 \times 10^{-1}$ | $1.1 \times 10^{-2}$ | $2.5 \times 10^{-3}$ | 4.5 | 0/0/0 | 1.533 | 1.526 | 30 | 514 |
| Experimental Example 21 | $1.8 \times 10^{-1}$ | $1.1 \times 10^{-2}$ | $2.1 \times 10^{-3}$ | 2.2 | 8/2/6 | - | - | - | - |
| Experimental Example 22 | $1.8 \times 10^{-1}$ | $1.1 \times 10^{-2}$ | $2.1 \times 10^{-3}$ | 2.6 | */*/* | - | - | - | - |
| Experimental Example 23 | $1.8 \times 10^{-1}$ | $1.1 \times 10^{-2}$ | $2.1 \times 10^{-3}$ | 2.7 | 3/*/ | - | - | - | - |

1) Molar fraction of hydrophilic monomer: molar fraction of hydrophilic monomers (HEMA, DMAA, and NVP) to all the polymerizable components
2) Molar fraction of crosslinkable monomer: molar fraction of crosslinkable monomers (BDDA and EDMA) to all the polymerizable components
3) *: Means that countless spots were present
4) -: Not measured
5) Two samples were measured in Experimental Example 23

EP 3 251 703 A1

[Table 3]

| Specimen | A × B[1] | Compressive load (mN)[2] | |
|---|---|---|---|
| | | Dry[3] | Wet[4] |
| Experimental Example 1 | $3.2 \times 10^{-3}$ | 630 | 382 |
| Experimental Example 3 | $3.0 \times 10^{-3}$ | 756 | 375 |
| Experimental Example 5 | $2.3 \times 10^{-3}$ | Overload | 349 |
| Experimental Example 6 | $3.2 \times 10^{-3}$ | Overload | 471 |
| Experimental Example 10 | $2.3 \times 10^{-3}$ | Overload | 453 |
| Experimental Example 11 | $3.9 \times 10^{-3}$ | Overload | 629 |
| Experimental Example 15 | $2.7 \times 10^{-3}$ | Overload | 226[5] |
| Experimental Example 16 | $3.6 \times 10^{-3}$ | Overload | 383[5] |
| Experimental Example 20 | $2.5 \times 10^{-3}$ | Overload | 311[5] |

1) A × B: (Molar fraction of hydrophilic monomer) × (Molar fraction of crosslinkable monomer)
2) Overload: More than the upper limit (787 mN) or did not bend
3) Dry: Dried at 80°C
4) Wet: Conditioned at 23°C and 50%RH (paper having a water absorption percentage of 5.1 mass% was put together)
5) Wet: Polymer having a water content of 65 mass% was put together

The present application claims priority from Japanese Patent Application No. 2015-015774 filed on January 29, 2015, the entire contents of which are incorporated herein by reference.

Industrial Applicability

The present invention may be used in applications that relate to intraocular lenses.

Reference Signs List

10 moisture-proof package, 11 Sterilized package, 12 Injector device, 14 Package insert, 20 Intraocular lens.

**Claims**

1. An intraocular lens material comprising a polymer produced by polymerizing a polymerizable composition including a hydrophilic monomer and a crosslinkable monomer,
   wherein an A×B value is more than $2.1 \times 10^{-3}$ and less than $3.6 \times 10^{-3}$, the A×B value being the product of a molar fraction A of the hydrophilic monomer and a molar fraction B of the crosslinkable monomer, the molar fraction B of the crosslinkable monomer is less than $2.4 \times 10^{-2}$, and the polymer has a refractive index n of 1.5 or more in a state of hydration.

2. The intraocular lens material according to Claim 1,
   wherein the molar fraction B of the crosslinkable monomer is less than $2.1 \times 10^{-2}$.

3. The intraocular lens material according to Claim 1 or 2,
   wherein the intraocular lens material has an elongation percentage of 290% or more.

4. The intraocular lens material according to any one of Claims 1 to 3,
   wherein the hydrophilic monomer includes one or more selected from a hydroxyl group-containing alkyl (meth)acrylate including an alkyl group having 1 to 20 carbon atoms, a (meth)acrylamide monomer, and an N-vinyl lactam.

5. The intraocular lens material according to any one of Claims 1 to 4,
   wherein the crosslinkable monomer includes one or more selected from butanediol di(meth)acrylate and ethylene glycol di(meth)acrylate.

6. The intraocular lens material according to any one of Claims 1 to 5,
wherein the polymerizable composition includes, as main components, an aromatic-ring-containing (meth)acrylate and an alkyl (meth)acrylate including an alkyl group having 1 to 20 carbon atoms.

7. A method for preserving an intraocular lens material, the method comprising:

enclosing the intraocular lens material according to any one of Claims 1 to 6 and a moistened humidity-controlling material in a moisture-proof package.

# Fig. 1

# Fig. 2

## INTERNATIONAL SEARCH REPORT

| International application No. |
|---|
| PCT/JP2016/052293 |

**A. CLASSIFICATION OF SUBJECT MATTER**

*A61L27/00*(2006.01)i, *C08F220/02*(2006.01)i, *G02C7/04*(2006.01)i

According to International Patent Classification (IPC) or to both national classification and IPC

**B. FIELDS SEARCHED**

Minimum documentation searched (classification system followed by classification symbols)
A61L27/00, C08F220/02, G02C7/04

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 11-56998 A  (Menicon Co., Ltd.),<br>02 March 1999 (02.03.1999),<br>paragraphs [0008], [0020], [0025] to [0036];<br>example 10<br>(Family: none) | 1,2,4-6<br>3,7 |

☒ Further documents are listed in the continuation of Box C.　　　☐ See patent family annex.

| | |
|---|---|
| * Special categories of cited documents:<br>"A" document defining the general state of the art which is not considered  to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>　06 April 2016 (06.04.16) | Date of mailing of the international search report<br>　19 April 2016 (19.04.16) |
|---|---|
| Name and mailing address of the ISA/<br>　Japan Patent Office<br>　3-4-3,Kasumigaseki,Chiyoda-ku,<br>　Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2016/052293 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br>Y | JP 2003-508605 A  (Alcon, Inc.),<br>04 March 2003 (04.03.2003),<br>paragraphs [0001], [0002], [0018], [0027],<br>[0029], [0034], [0035]; experimental example 9<br>& WO 2001/018079 A1<br>page 1, lines 4 to 16; page 4, line 28 to page<br>5, line 2; page 6, line 30 to page 7, line 3;<br>page 7, lines 15 to 31; page 8, line 25 to page<br>9, line 8; example 9<br>& EP 1210381 A1          & AU 7070500 A<br>& BR 13779 A            & CA 2381177 A1<br>& CN 1371394 A          & AR 25572 A1 | 1-6<br>3,7 |
| Y | JP 2008-525155 A  (Bausch & Lomb Inc.),<br>17 July 2008 (17.07.2008),<br>paragraph [0014]<br>& US 2006/0142780 A1<br>paragraph [0038]<br>& WO 2006/071597 A2      & EP 1833423 A2<br>& CA 2593074 A1          & KR 10-2007-0092246 A<br>& CN 101094624 A        & AU 2005322301 A1<br>& ES 2460890 T3 | 7 |
| Y | JP 2004-357644 A  (Uni-Charm Corp.),<br>24 December 2004 (24.12.2004),<br>paragraphs [0002] to [0004], [0012], [0018],<br>[0039]<br>(Family: none) | 7 |
| Y | JP 2008-519890 A  (Multisorb Technologies,<br>Inc.),<br>12 June 2008 (12.06.2008),<br>paragraphs [0004], [0007]<br>& US 2006/0097223 A1<br>paragraphs [0004], [0007]<br>& WO 2006/052748 A2      & EP 1812526 A2<br>& CA 2584811 A1          & KR 10-2007-0084053 A<br>& CN 101437917 A        & MX 2007005514 A<br>& RU 2007121666 A        & KR 10-1022952 B1<br>& CN 103212278 A | 7 |
| Y | JP 2008-61862 A  (Hisamitsu Pharmaceutical Co.,<br>Inc.),<br>21 March 2008 (21.03.2008),<br>paragraphs [0006], [0008], [0014], [0015],<br>[0017], [0018]<br>(Family: none) | 7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11056998 A
- JP 2015015774 A